# EUROPEAN PATENT SPECIFICATION

(11) **EP 0 859 952 B1**
(45) Date of publication and mention of the grant of the patent: **09.04.2003**
(21) Application number: 96937212.7
(22) Date of filing: 17.10.1996
(51) Int. Cl.: G01N 21/35, G01N 33/28

(54) **METHOD FOR PREDICTING A PHYSICAL PROPERTY OF A RESIDUAL HYDROCARBONACEOUS MATERIAL**
METHODE ZUR VORHERSAGE EINER PHYSIKALISCHEN EIGENSCHAFT EINES RESTKOHLENWASSERSTOFFHALTIGEN MATERIALS
PROCEDE DE PREDICTION D'UNE PROPRIETE PHYSIQUE D'UN MATERIAU HYDROCARBONE RESIDUEL

(30) Priority: 18.10.1995 EP 98307410; 11.03.1996 EP 96301647
(43) Date of publication of application: 26.08.1998
(73) Proprietor: SHELL INTERNATIONALE RESEARCH MAATSCHAPPIJ B.V., 2596 HR Den Haag (NL)
(72) Inventor: VAN DOORN, Ronald, NL-1031 CM Amsterdam (NL); HOOYMAN-SPAARGAREN, Froukje, Haasje, NL-1031 CM Amsterdam (NL); NEUGEBAUER, Ronald, Johan, NL-1031 CM Amsterdam (NL); SMEETS, Louis, Marie, NL-1031 CM Amsterdam (NL)
(86) International application number: EP9604599
(87) International publication number: WO97014953

(56) References cited:
- EP-A- 0 285 251
- EP-A- 0 304 232
- WO-A-94/08226
- WO-A-94/09362

## Description

The present invention relates to a method for predicting a physical property of a residual hydrocarbonaceous material by correlation of its (near) infrared spectrum to the physical property.

The use of (near) infrared spectroscopy to control processes for the preparation of petroleum products is known for instance from "Hydrocarbon Processing", February 1995, pages 86-92. The processes described in said document include the preparation of gasolines and gas oils by the controlled blending of various components. The quality of the final product is determined on-line using a Fourier transform-type of spectrometer which is connected to a computer. In this way the use of blend tables can advantageously be avoided.

Another type of process widely applied in petroleum industry, in respect of which it would be highly advantageous to control continuously the product quality by means of (near) infrared spectroscopy, is the preparation of bitumen compositions by blending various streams of different grades of bitumen. Attempts to use (near) infrared spectroscopy for controlling the quality of bitumen compositions have, however, been rather disappointing so far, which can most likely be attributed to the very heavy components of which bituminous materials are built up.

In this respect reference is made to "Rapid Prediction and Evaluation of Bitumen Properties by Near Infrared Spectroscopy", G. Svechinsky and I. Ishia, which paper was presented at the Third Annual Meeting of RILEM Committee TC PBM-152, Madrid, Spain, June 1995. In said paper the use has been described, without any details, of the reflection of near infrared radiation for characterization and prediction of different bitumen parameters.

Object of the present invention is to provide an advanced method for predicting a physical property of a crude oil residues residual fuel oils and bituminous materials using near infrared spectra.

The present invention therefore provides a method for predicting a physical property of a crude oil residue, a residual fuel oil or a bituminous material comprising the steps of:
a) selecting a set of crude oil residues, residual fuel oils or bituminous materials of different quality;
b) determining a physical property of the crude oil residues, residual fuel oils or bituminous materials by conventional measurement;
c) measuring the near infrared spectra of the crude oil residues, residual fuel oils or bituminous materials;
d) selecting in the spectral region a range of wavelengths, and using the absorbance values measured at these wavelengths as an input for multivariate statistical analysis or a neural network;
e) correlating the absorbance values obtained with the physical property as determined under b) by means of multivariate statistical analysis or a neural network and generating a predictive model; and subsequently
f) applying this predictive model to near infrared spectra, taken under the same conditions, for a crude oil residue, a residual fuel oil or a bituminous material of an unknown physical property, thus providing the physical property of the unknown crude oil residue, a residual fuel oil or a bituminous material, characterized in that the measurement of part c) is carried out in a transmission cell.

By means of the above method it is for instance possible to control continuously the quality of a hydrocarbonaceous feedstock and/or the product derived therefrom.

According to the present invention the near infrared spectra of a relatively large set of residual hydrocarbonaceous materials (suitably at least 10, preferably at least 50) of different quality are measured.

The number of hydrocarbonaceous materials of different quality in the set is important since this determines the generality and applicability of any subsequent statistical predictive pool.

The light from the (near) infrared region has wavelengths in the range of from 1000 to 10,000 nm, preferably in the range of from 1500 to 3000 nm, more preferably in the range of from 1640 to 2630 nm or one or more selected intervals thereof.

The spectra obtained can be analysed, together with determinations of the physical property by conventional measurements, using multivariate statistical analysis techniques such as Partial Least Squares, Multiple Linear Regression, Reduced Rank Regression, Principal Component Analysis and the like, or neural networks.

The above-mentioned multivariate statistical techniques and neural networks are as such known to those skilled in the art and will therefore not be described in detail.

Suitably, the absorbance values are measured at a large number of the wavelengths in the spectral region. Suitably, the absorbance values are measured at the whole range of wavelengths in the spectral region or at one or more selected intervals thereof.

Subsequently a predictive model is generated that can be applied to the (near) infrared spectra, taken under the same conditions, for residual hydrocarbonaceous materials of an unknown physical property.

Correlation of the absorbance values with the physical property of the residual hydrocarbonaceous materials as determined under b) is done by known techniques mentioned before such as multiple linear regression or partial least squares regression.

The residual hydrocarbonaceous materials of which a physical property can be determined in accordance with the present invention comprise for instance crude oil residues, residual fuel oils and bituminous materials.

Crude oil residues may consist of straight run residues such as long (atmospheric) and short (vacuum) residues, processed residue streams such as thermally cracked, hydrocracked or catalytically cracked residues. Residual fuel oils may consist of residues and any known diluent streams such as any refinery stream to influence residue properties, and may contain any known additive such as stabilising or emulsifying agents.

Suitable bituminous materials include naturally occurring bitumens or derived from a mineral oil. Also blends of various bituminous materials can be analysed. Examples of suitable bituminous materials include distillation or "straight-run bitumens", cracked residues, polymer-modified bitumens, precipitation bitumens, e.g. propane bitumens, blown bitumens, e.g. catalytically blown bitumen, and mixtures thereof.

Other suitable bituminous materials include mixtures of one or more of these bitumens with extenders (fluxes) such as petroleum extracts, e.g. aromatic extracts, distillates or residues, or with oils. The bituminous materials to be analysed may contain any emulsifying agent known in the art.

The above-mentioned residual hydrocarbonaceous materials to be analysed by means of (near) infrared spectroscopy have suitably a temperature of at least 50°C, preferably a temperature of at least 100°C.

The physical properties to be determined include properties such as penetration (PEN), softening point, density, viscosity, flash point, storage and handling stability, compatibility, and chemical composition related properties such as aromaticity, C7 asphaltenes content, wax content, paraffin content, volatility and retained PEN (after RTFOT (Rolling thin film oven test)), microcarbon residue, Conradson carbon residue and feedstock assessment parameters.

In accordance with the present invention two or more physical properties, e.g. softening point and PEN, of a residual hydrocarbonaceous material can be determined simultaneously.

The present invention will now be illustrated by means of the following Example.

### Example 1

A set of 72 bituminous materials of different quality was selected. The samples were all rated for penetration and softening point values using the ASTM D 5 and ASTM D 36 methods respectively. The samples were subsequently measured in the near infrared region having wavelenghts in the range of from 1640 to 2630 nm. Samples of the bituminous materials were in turn contained in a transmission cell at a temperature of 200 °C and the near infrared spectra were recorded. A predictive model was generated using partial least squares regression as explained before. The standard deviations of prediction are 3 dmm for penetration in the range 20-140 dmm, and 0.7 °C for softening point in the range 42-62 °C, as determined by the leave-one-out cross-validation method.

### Example 2

A set of 75 thermally cracked residues of different quality was selected. The samples were all rated for Micro Carbon Residue content and viscosity using the ASTM D-4530-93 and ASTM D-445-94 methods respectively. The samples were subsequently measured in the near infrared region having wavelengths in the range of from 1640 to 2630 nm. Samples of the residues were in turn contained in a transmission cell at a temperature of 200 °C and the near infrared spectra were recorded. A predictive model was generated using partial least squares regression as explained before. The standard deviations of predictions are 0.4 %w/w for Micro Carbon Residue content in the range of 24.3 - 35.2 %w/w, and 6.5% for viscosity in the range of 65 - 554 cSt, as determined by the leave-one-out cross validation method.

It will be clear from the foregoing that the present invention provides a very attractive method for predicting physical properties of residual hydrocarbonaceous materials.

## Claims

1. A method for predicting a physical property of a crude oil residue, a residual fuel oil or a bituminous material, which method comprises the steps of:
a) selecting a set of crude oil residues, residual fuel oils or bituminous materials of different quality;
b) determining a physical property of the crude oil residues, residual fuel oils or bituminous materials by conventional measurement;
c) measuring the near infrared spectra of the crude oil residues, residual fuel oils or bituminous materials;
d) selecting in the spectral region a range of wavelengths, and using the absorbance values measured at these wavelengths as an input for multivariate statistical analysis or a neural network;
e) correlating the absorbance values obtained with the physical property as determined under b) by means of multivariate statistical analysis or a neural network and generating a predictive model; and subsequently
f) applying this predictive model to near infrared spectra, taken under the same conditions, for a crude oil residue, a residual fuel oil or a bituminous material of an unknown physical property, thus providing the physical property of the unknown crude oil residue, residual'fuel oil or bituminous material, **characterized in that** the measurement of part c) is carried out in a transmission cell.

2. A method according to claim 1, wherein the method is for predicting a physical property of a bituminous material, which method comprises the steps of:
a) selecting a set of bituminous materials of different quality;
b) determining a physical property of the bituminous materials by conventional measurement;
c) measuring the near infrared spectra of the bituminous materials;
d) selecting in the spectral region a range of wavelengths, and using the absorbance values measured at these wavelengths as an input for multivariate statistical analysis or a neural network;
e) correlating the absorbance values obtained with the physical property as determined under b) by means of multivariate statistical analysis or a neural network and generating a predictive model; and subsequently
f) applying this predictive model to near infrared spectra, taken under the same conditions, for a bituminous material of an unknown physical property, thus providing the physical property of the unknown bituminous material.

3. The method according to claim 1 or claim 2, wherein the near infrared region has wavelengths in the range of from 1000 to 10,000 nm.

4. The method according to claim 3, wherein the wavelengths range of from 1500 to 3000 nm.

5. The method according to any one of claims 1 to 4, wherein the set of crude oil residues, residual fuel oils or bituminous materials is at least 50.

## Patentansprüche

1. Verfahren zur Vorhersage einer physikalischen Eigenschaft eines Rohölrückstandes, eines Rückstandsheizöls oder eines bituminösen Materials, das die folgenden Stufen umfaßt:
a) Auswählen eines Satzes von Rohölrückständen, Rückstandsheizölen oder bituminösen Materialien unterschiedlicher Qualität;
b) Bestimmen einer physikalischen Eigenschaft der Rohölrückstände, Rückstandsheizöle oder bituminösen Materialien durch konventionelle Messung;
c) Messen der Nahinfrarotspektren der Rohölrückstände, Rückstandsheizöle oder bituminösen Materialien;
d) Auswählen eines Wellenlängenbereiches in dem Spektralbereich und Anwenden der bei diesen Wellenlängen gemessenen Absorptionswerte als Eingang für eine-multivariantenstatistische Analyse oder ein neurales Netzwerk;
e) Korrelieren der erhaltenen Absorptionswerte mit der physikalischen Eigenschaft, wie unter b) bestimmt, mittels multivariantenstatistischer Analyse oder einem neuralen Netzwerk und Generieren eines Vorhersagemodells; und anschließend
f) Anwenden dieses Vorhersagemodells auf Nahinfrarotspektren, die unter den gleichen Bedingungen genommen wurden, auf einen Rohölrückstand, ein Rückstandsheizöl oder ein bituminöses Material mit einer unbekannten physikalischen Eigenschaft, wodurch die physikalische Eigenschaft des unbekannten Rohölrückstandes, eines Rückstandsheizöls oder eines bituminösen Materials geliefert wird, **dadurch gekennzeichnet, daß** die Messung von Teil c) in einer Transmissionszelle vorgenommen wird.

2. Verfahren nach Anspruch 1, worin das Verfahren zur Vorhersage einer physikalischen Eigenschaften eines bituminösen Materials dient, welches Verfahren die folgenden Stufen umfasst:
a) Auswählen eines Satzes von bituminösen Materialien unterschiedlicher Qualität;
b) Bestimmen einer physikalischen Eigenschaft der bituminösen Materialien durch konventionelle Messung;
c) Messen der Nahinfrarotspektren der bituminösen Materialien;
d) Auswählen eines Wellenlängenbereiches in dem Spektralbereich und Anwenden der bei diesen Wellenlängen gemessenen Absorptionswerte als Eingang für eine multivariantenstatistische Analyse oder ein neurales Netzwerk;
e) Korrelieren der erhaltenen Absorptionswerte mit der physikalischen Eigenschaft, wie unter b) bestimmt, mittels multivariantenstatistischer Analyse oder einem neuralen Netzwerk und Generieren eines Vorhersagemodells; und anschließend
f) Anwenden dieses Vorhersagemodells auf Nahinfrarotspektren, die unter den gleichen Bedingungen genommen wurden, auf ein bituminöses Material mit einer unbekannten physikalischen Eigenschaft, wodurch die physikalische Eigenschaft des unbekannten bituminösen Materials geliefert wird.

3. Verfahren nach Anspruch 1 oder 2, worin der Naheinfrarotbereich Wellenlängen im Bereich von 1.000 bis 10.000 nm aufweist.

4. Verfahren nach Anspruch 3, worin die Wellenlängen im Bereich von 1.500 bis 3.000 nm liegen.

5. Verfahren nach einem der Ansprüche 1 bis 4, worin der Satz aus Rohölrückständen, Rückstandsheizölen oder bituminösen Materialien wenigstens 50 ist.

## Revendications

1. Procédé de prédiction d'une propriété physique d'un résidu d'huile brute, d'une huile combustible résiduelle ou d'une matière bitumineuse, lequel procédé comprend les étapes suivantes :
a) la sélection d'une série de résidus d'huile brute, d'huiles combustibles résiduelles ou des matières bitumineuses de différente qualité;
b) la détermination d'une propriété physique des résidus d'huile brute, huiles combustibles résiduelles ou matières bitumineuses par une mesure conventionnelle;
c) la mesure des spectres infrarouges proches des résidus d'huile brute, huiles combustibles résiduelles ou matières bitumineuses;
d) la sélection dans la région spectrale d'une gamme de longueurs d'onde, et l'utilisation des valeurs d'absorption mesurées à ces longueurs d'onde comme entrée pour une analyse statistique multivariée ou un réseau neuronal;
e) la corrélation des valeurs d'absorption obtenues avec la propriété physique telle que déterminée sous b) au moyen d'une analyse statistique multivariée ou d'un réseau neuronal et la génération d'un modèle prédictif; et ensuite
f) l'application de ce modèle prédictif à des spectres infrarouges proches, pris sous les mêmes conditions, pour un résidu d'huile brute, une huile combustible résiduelle ou une matière bitumineuse d'une propriété physique inconnue, en obtenant ainsi la propriété physique du résidu d'huile brute, de l'huile combustible résiduelle ou de la matière bitumineuse inconnu,
**caractérisé en ce que** la mesure de la partie c) est réalisée dans une cellule de transmission.

2. Procédé suivant la revendication 1, dans lequel le procédé est utilisé pour la prédiction d'une propriété physique d'une matière bitumineuse, lequel procédé comprend les étapes suivantes :
a) la sélection d'une série de matières bitumineuses de différente qualité;
b) la détermination d'une propriété physique des matières bitumineuses par une mesure conventionnelle;
c) la mesure des spectres infrarouges proches des matières bitumineuses;
d) la sélection dans la région spectrale d'une gamme de longueurs d'onde, et l'utilisation des valeurs d'absorption mesurées à ces longueurs d'onde comme entrée pour une analyse statistique multivariée ou un réseau neuronal;
e) la corrélation des valeurs d'absorption obtenues avec la propriété physique telle que déterminée sous b) au moyen d'une analyse statistique multivariée ou d'un réseau neuronal et la génération d'un modèle prédictif; et ensuite
f) l'application de ce modèle prédictif à des spectres infrarouges proches, pris sous les mêmes conditions, pour une matière bitumineuse d'une propriété physique inconnue, en obtenant ainsi la propriété physique de la matière bitumineuse inconnue.

3. Procédé suivant l'une ou l'autre des revendications 1 et 2, dans lequel la région infrarouge proche a des longueurs d'onde allant de 1.000 à 10.000 nm.

4. Procédé suivant la revendication 3, dans lequel les longueurs d'onde vont de 1.500 à 3.000 nm.

5. Procédé suivant l'une quelconque des revendications 1 à 4, dans lequel la série de résidus d'huile brute, d'huiles combustibles ou résiduelles ou de matières bitumineuses est d'au moins 50.
